# EUROPEAN PATENT APPLICATION

(11) **EP 1 681 892 A1**
(43) Date of publication of application: **19.07.2006**
(21) Application number: 06000947.9
(22) Date of filing: 17.01.2006
(51) Int. Cl.: H04Q 7/38

(54) **Method for automatic frequency band selection in multi-band supported mobile station**

(30) Priority: 18.01.2005 KR 2005004683
(71) Applicant: Samsung Electronics Co., Ltd., Suwon-si, Gyeonggi-Do (KR)
(72) Inventor: Hwang, Sun-Ho, c/o Samsung Electronics Co., Ltd., Suwon-si, Gyeonggi-do (KR)
(74) Representative: Grünecker, Kinkeldey, Stockmair & Schwanhäusser Anwaltssozietät

(57) **Abstract**

Disclosed is an automatic frequency band selection method in a multi-band supported mobile station. In the method, the mobile station checks a Mobile Country Code (MCC) and a Mobile Network Code (MNC) from system information when international roaming has occurred, selects a frequency band corresponding to the MCC and MNC checked from a frequency band information table as a search frequency band, the frequency band information table storing in advance information for frequency bands used according to each country/network, and searches for the selected frequency band, thereby performing network synchronization. According to the automatic frequency band selection method, frequency search time can be reduced, network registration can be performed more quickly when international roaming has occurred, and unnecessary operations can be omitted. Consequently, it is possible to prevent the battery from being wasted.

## Description

The present invention relates to frequency band selection for a mobile communication terminal, and more particularly to an automatic frequency band selection method in a multi-band supported mobile station, which can be applied to a Global System for Mobile communication (GSM)/General Packet Radio Service (GPRS) mobile station. The present invention also relates to a corresponding apparatus.

A GSM system is a digital mobile phone system classified as a cellular system, which is widely used in Europe and other regions. A GPRS developed based on this GSM scheme is a more advanced packet-based mobile communication service.

Because many GSM network operators have entered into a roaming agreement with operators in other countries, GSM provides a service in which domestic users can continue to use their own mobile phones when they travel abroad. Herein, a GSM/GPRS mobile station has been designed to usually support a multi-band (more than tri-band) range in order to operate in the different frequency bands of each country.

In order to select a frequency band for the multi-band supported GSM/GPRS mobile station, the following two schemes are usually used. One is the most used scheme in which a user can select a supportable frequency band from plural frequency band choices on a menu, which are previously provided in the mobile station. Accordingly, when a user has moved to a country supporting a new frequency band, the user can optionally select the frequency band. For example, because most countries in Europe have used an Extended GSM (EGSM)/Digital Communication System (DCS) band, and the USA and South America have used 850 (GSM 850 MHz)/Personal Communication Service (PCS) band, a user can select the 850/PCS band by using the menu of a mobile station when the user with a mobile station supporting an EGSM/DCS/PCS/850 band in Europe has moved to the USA. In this case, the user must know the frequency bands in advance used in a country in which the user is located.

The other scheme is a scheme in which a mobile station can automatically search all frequency bands and select a band corresponding to a country in which the mobile station is currently located. In this case, the mobile station must search for the corresponding frequencies from among all bands in order to find the frequency bands of the corresponding country.

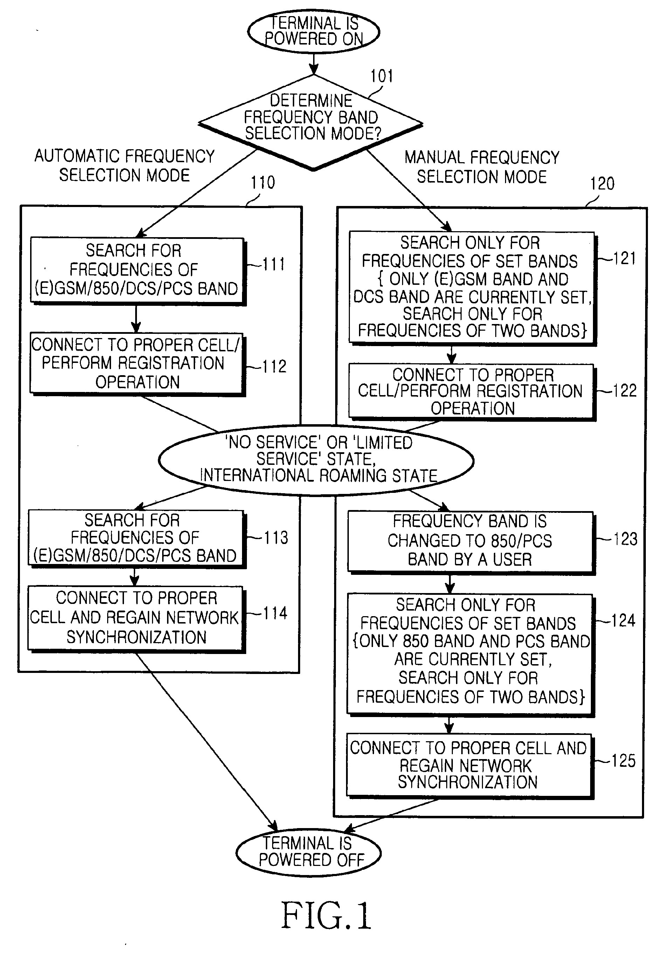

FIG. 1 is a flow diagram schematically illustrating a conventional frequency band selection operation for a multi-band supported mobile station. Referring to FIG. 1, when the mobile station is powered on, the mobile station determines the currently set frequency band selection mode in step 101. When the currently set mode is a manual frequency selection mode, the mobile station performs an operation according to the manual frequency selection mode in step 120. However, when the currently set mode is an automatic frequency selection mode, the mobile station performs an operation according to the automatic frequency selection mode in step 110.

Hereinafter, the operation in step 120 according to the manual frequency selection mode will be described. In step 121, the mobile station searches only for frequencies of currently set bands. For example, when only an (E)GSM band and a DCS band are currently set, the mobile station searches only for frequencies of the two bands. In step 122, the mobile station connects to a proper cell according to the searched frequencies and performs a registration operation, etc, thereby performing network synchronization. Herein, when a corresponding frequency is not normally searched, that is, when the mobile station is located in a 'No Service' area or a 'Limited Service' area, or when international roaming has occurred, a frequency band selection operation by a user is performed in step 123. For example, a user can change the (E)GSM band and the DCS band into an 850 band and a PCS band. In step 124, the mobile station searches only for frequencies of the currently set bands (e.g., the 850 band and the PCS band). Then, in step 125, the mobile station connects to a proper cell and recovers the network synchronization.

In the meantime, when a user does not set a frequency band according to an actual network in the aforedescribed process, the mobile station continuously remains in a 'No Service' state or a 'Limited Service' state.

Hereinafter, the operation in step 110 according to the automatic frequency selection mode will be described. In step 111, the mobile station searches for all frequencies of (E)GSM/850/DCS/PCS band. In step 112, the mobile station connects to a proper cell according to the searched frequencies and performs a registration operation, etc., thereby performing network synchronization. Herein, when network synchronization is impossible, that is, when the mobile station is located in a 'No Service' area or a 'Limited Service' area, or when international roaming has occurred, the mobile station searches for all frequencies of (E)GSM/850/DCS/PCS bands again in step 113. Then, in step 114, the mobile station connects to a proper cell and recovers the network synchronization.

The operations of the manual and the automatic frequency selection mode in the multi-band supported mobile station can be performed by the operation as illustrated in FIG. 1. These operations of the manual and the automatic frequency selection mode are performed until the mobile station is powered off.

Further, when the frequency band must be manually selected as described above, the user must know the corresponding frequency band information of a country before the user moves. If the user does not know the information, the user must check the information through a separate manual, etc., and select a frequency band in the manual mode.

If a frequency band is selected in the automatic mode, there may occur a difference in algorithms executed in the mobile station, but a case where corresponding frequencies of all bands must be searched may occur in order to determine frequency bands of a corresponding country. In this case, the following expected problems may occur. That is, if the original mobile station is powered on after movement to another country or the mobile station moves while in a state where the mobile station is not powered off, it may take a long time to become registered in a corresponding network in the country having different bands, and network synchronization must be performed for unnecessary frequencies.

For example, as illustrated in the following Table 1 showing an Absolute Radio Frequency Channel Number (ARFCN) and the number of frequencies according to each frequency band, a mobile station currently supporting four bands has a total of 971 frequencies to be searched. This figure is obtained by summing up 174 frequencies of an EGSM band, 374 frequencies of a DCS band, 299 frequencies of a PCS band, and 124 frequencies of a GSM 850 band.

**Table 1**

| Frequency band | ARFCN | Number of frequencies |
|---|---|---|
| 850 band (850 MHz) | ARFCN 128 ~ ARFCN 251 | 124 |
| (E)GSM band (900 MHz) | ARFCN 0 ~ ARFCN 124, | 174 |
| | ARFCN 975 ~ ARFCN 1023 | |
| DCS band (1800 MHz) | ARFCN 512 ~ ARFCN 885 | 374 |
| PCS band (1900 MHz) | ARFCN 512 ~ ARFCN 810 | 299 |

In Table 1, the PCS band has a different number of frequencies from that of the DCS band, but the used ARFCN number overlaps in the two bands (i.e., two do not exist in one network). Accordingly, even though the number of frequencies is reduced, there exist at least 500 frequencies to be searched. Further, when the frequency is not a Broadcast Control Channel Carrier (BCCH) for network synchronization, about 0.5 second at maximum is currently required to search each frequency. Accordingly, when taking these conditions into consideration, it may be impossible to perform network synchronization for a corresponding frequency for a maximum of 10 minutes. Currently, many frequencies as described above do not exist in most cases. However, in the worst case, this situation may occur. Moreover, the search may be necessary not only when the mobile station is first powered on but also when the mobile station is located in a 'No Service' area or a 'Limited Service' area, resulting in a search of all frequencies of a corresponding frequency band and performance of synchronization of the searched frequencies. Due to these problems, it may take a long time to acquire normal networking, and battery consumption of the multi-band supported mobile station in searching frequencies may increase as compared with a general single-band mobile station.

Accordingly, the present invention has been made to solve the above-mentioned problems occurring in the prior art.

It is the object of the present invention to provide an automatic frequency band selection method in a multi-band supported mobile station, which can reduce frequency search time.

This object is solved by the subject matter of the independent claims.

Preferred embodiments are defined in the dependent claims.

It is an aspect of the present invention to provide an automatic frequency band selection method in which a network registration can be performed quickly and unnecessary operations can be omitted when international roaming has occurred due to movement between countries, thereby preventing the battery from being wasted.

It is a further aspect of the present invention to provide an apparatus that is adapted to perform an automatic frequency bond selection method as defined above.

In order to accomplish the aforementioned object, according to one aspect of the present, there is provided an automatic frequency band selection method in a multi-band supported mobile station. According to the method, a table (database) capable of managing frequency bands according to each country is previously provided in the mobile station in order to solve the problems in the prior art, and frequency bands of countries to which a user frequently travels are learned and stored in the mobile station. The identification of the frequency bands can be performed through a local frequency band search using software and hardware in the mobile station, or from system information sent from a network to the mobile station.

The present invention will be more apparent from the following detailed description, taken in conjunction with the accompanying drawings, in which:
FIG. 1 is a flow diagram schematically illustrating a conventional frequency band selection operation for a multi-band supported mobile station;
FIG. 2 is a block diagram schematically illustrating the construction of a multi-band supported mobile station to which the present invention is applied; and
FIG. 3 is a flow diagram illustrating an automatic frequency band selection operation in a multi-band supported mobile station according to one embodiment of the present invention.

Hereinafter, preferred embodiments according to the present invention will be described with reference to the accompanying drawings. In the following description, detailed elements are provided for helping the general understanding of the present invention, it will be understood by those skilled in the art that these elements may be modified within the scope of the present invention.

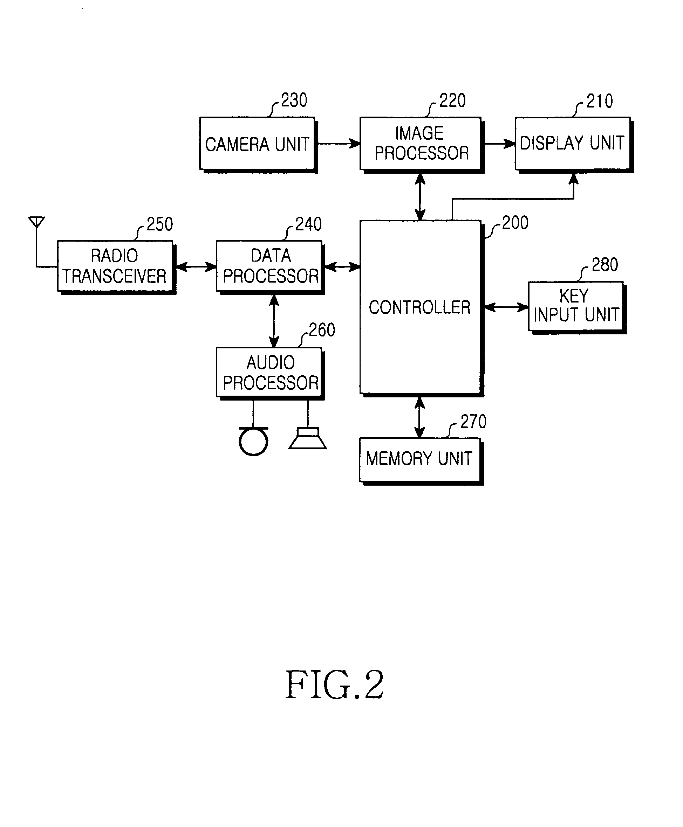

FIG. 2 is a block diagram schematically illustrating the construction of a multi-band supported mobile station to which the present invention is applied. Referring to FIG. 2, a radio transceiver 250 receives frequency signals on radio channels of a predetermined frequency band through an antenna under the control of a controller 200. A data processor 240 down-converts the band of the received frequency signals, classifies types of data, and outputs the classified data to the controller 200. The data output to the controller 200 from the radio transceiver 250 includes text data or paging signals and signaling signals received through a paging channel.

A key input unit 280 includes a plurality of number keys and function keys for performing various functions, which outputs electrical signals of key data from a user's key input to the controller 200. An audio processor 260 is generally comprised of a vocoder and operates under the control of the controller 200. The audio processor 260 converts coded voice data received from the radio transceiver 250 into electrical voice signals through decoding, and outputs the electrical voice signals to a speaker. Then, the speaker converts the received electrical voice signals to audible sounds and outputs the audible sounds. Further, the audio processor 260 codes electrical voice signals received from a microphone and outputs the coded signals to the radio transceiver 250.

A camera unit 230 includes a camera sensor for photographing image data and converting photographed optical signals into electrical signals, and a signal processor for converting analog image signals photographed by the camera sensor into digital data. An image processor 220 performs a function of generating screen data for displaying the image signals output from the camera unit 230 on display unit 210. In terms of hardware, a memory unit 270 is comprised of a Read Only Memory (ROM) for storing operating programs, an Electrically Erasable Programmable Read-Only Memory (EEPROM), a Random Access Memory (RAM), etc. In terms of software, this memory unit 270 may be comprised of a program memory and a data memory, which stores various information required for controlling the operations of the mobile communication terminal. Further, the memory unit 270 stores an information table for frequency bands used according to each country according to the present invention.

The controller 200 controls the components and directs the general operations of the mobile communication terminal. In particular, the controller 200 performs an automatic frequency band selection operation with reference to the information table stored in the memory unit 270, according to the present invention.

In the present invention, whether international roaming has occurred is determined using a Mobile Country Code (MCC) obtained from system information sent from a network. The system information includes a three digit MCC and a two digit Mobile Network Code (MNC) as shown in Table 2.

**Table 2**

| | |
|---|---|
| MCC | MNC |

Herein, it is possible to check frequency bands of a corresponding network through a field for reporting the frequency bands of the corresponding network in the system information sent from the network. This information does not indicate all bands, but represents information on a DCS band and a PCS band using overlapping frequency numbers. Referring to the GSM Spec. 04. 18v8. 21. 0-10. 5. 2. 32 standard, a value of "Band Indicator" in 'System Information 1' received from the network is checked, so that it is possible to determine if a frequency band currently used in the network is a DCS band or a PCS band. That is, the "Band Indicator" is one bit information. When the corresponding bit is 0, the current frequency band is the DCS band. When the corresponding bit is 1, the current frequency band is the PCS band.

According to the present invention, when the mobile station band selection is set as an automatic selection, whether international roaming has occurred is checked. The frequency band information supported by the corresponding network is obtained from the system information sent from the network or the corresponding frequency band is obtained through a local search by hardware in the mobile station, and the obtained frequency band information is stored as the information table as shown in Table 3.

**Table 3**

| MCC/MNC | BAND | MCC/MNC | BAND |
|---|---|---|---|
| 001 01 | (E)GSM/DCS | 262 01 | (E)GSM |
| 001 02 | DCS Only | 003 01 | (E)GSM/DCS |
| 00201 | 850/PCS | 00403 | 850/PCS |
| 01002 | 850/PCS | 22201 | DCS Only |
| 03001 | PCS Only | 00501 | PCS Only |

Further, in the present invention, new frequency band information is updated to the mobile station. For example, in the USA, only the PCS band has been supported in the past, but providers have recently constructed a network in order to support the 850 MHz band. This is because an updating for new band information is necessary.

In a case where a user of a mobile communication terminal moves from one country to another country and turns on the power of the mobile station, when a manual search has been set for frequency bands in the prior art, frequency bands of a corresponding country are searched according to a frequency band selection, or only frequencies of a preset band are searched in the mobile station. However, when an automatic search has been set for the frequency bands, an unnecessary synchronization operation is performed for frequencies which are not used in the corresponding country. This may cause unnecessary search time and electric power consumption. In order to prevent these problems from occurring, the present invention recognizes the occurrence of roaming in another country by means of the system information received from the network, reads the frequency bands of the corresponding country from the corresponding frequency band information table as shown in Table 3, and automatically selects the proper frequency band for the country. Herein, when the user moves to another country again, the mobile station searches only for the currently selected frequency band. Accordingly, when the mobile station is continuously in a 'No Service' state for a predetermined time period (e.g., two minutes), the mobile station searches for all frequencies again. Herein, when the mobile station does not search for a corresponding frequency and remains in the 'No Service' state, it is possible to allow the mobile station to perform a repetition process for searching only for the originally selected frequency band again. Further, when frequency bands for a new country have been searched through a search for all frequencies, the mobile station according to the present invention is designed to select frequency bands again. For example, in a state where the GSM/DCS band has been set for a country having a code of '001 01' in Table 3, when the mobile station has moved to a new a country having a code of '002 01', the frequency band is automatically selected for the 850/PCS band from the GSM/DCS band by means of the information of Table 3 in the mobile station. Therefore, only frequencies for these two bands are searched. Further, when new frequencies having not existed in the existing frequency band information table in the mobile station have been searched during the search for all frequencies, a corresponding band of the searched frequencies is updated to the frequency band information table.

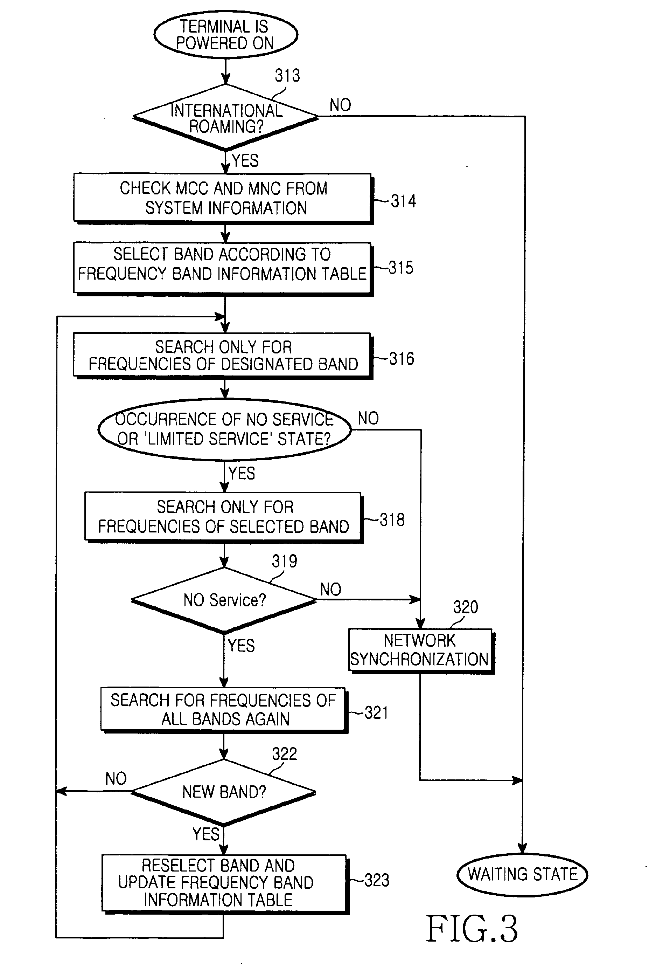

FIG. 3 is a flow diagram illustrating the automatic frequency band selection operation in the multi-band supported mobile station according to one embodiment of the present invention. Referring to FIG. 3, in a state where the mobile station has been powered on, the occurrence of international roaming is checked in step 313.

In step 314, the mobile station checks the MCC and the MNC from the system information provided from the network. In step 315, the mobile station selects a band according to a corresponding frequency band information table and proceeds to step 316. In this case, when information for a band designated in advance according to the corresponding MCC and MNC does not exist in the frequency band information table, all frequency bands are searched. Then, the searched band may be designated as a band according to the MCC and MNC and stored in the frequency band information table. In step 316, the mobile station searches only for frequencies of the designated band.

Further, after the band is selected as described in step 315, when the mobile station is powered on, etc., the mobile station searches only for the frequencies of the selected band as described in step 316.

Herein, when network synchronization is impossible, that is, when a 'No Service' state or a 'Limited Service' state has occurred, step 318 is performed in order to search only for the frequencies of the selected band. When the mobile station searches only for the frequencies of the selected band, it is determined if the mobile station does not search for a corresponding frequency and still remains in the 'No Service' state, in step 319. When the mobile station is not in the 'No Service' state, the mobile station connects to a proper cell and recovers the network synchronization in step 320. However, when the mobile station is in the 'No Service' state, the mobile station searches for frequencies of all (E)GSM/850/DCS/PCS bands in step 321. As a result of this search, whether new frequencies having not existed in the existing frequency band information table are searched is determined in step 322. When the new band is searched, step 323 is performed. In step 323, the mobile station reselects the band and updates the corresponding frequency band to the frequency band information table.

The operations in the manual/automatic frequency selection mode in the multi-band supported mobile station according to the present invention can be performed by the operation as shown in FIG. 3. These operations in the manual/automatic frequency selection mode are performed until the mobile station is powered off.

According to an automatic frequency band selection scheme in a multi-band supported mobile station of the present invention as described above, frequency search time can be reduced, network registration can be performed more quickly when international roaming has occurred, and unnecessary operations can be omitted. Consequently, it is possible to prevent the battery from being wasted.

While the present invention has been shown and described with reference to certain preferred embodiments thereof, it will be understood by those skilled in the art that various changes in form and details may be made therein without departing from the scope of the invention as defined by the appended claims.

## Claims

1. An automatic frequency band selection method in a multi-band supported mobile station, the method comprising the steps of:
checking a Mobile Country Code, MCC, and a Mobile Network Code, MNC, from system information when international roaming has occurred;
selecting a frequency band corresponding to the MCC and MNC checked from a frequency band information table as a search frequency band, the frequency band information table storing information for frequency bands used according to each country/network;
searching for the selected frequency band; and
performing network synchronization.

2. The method as claimed in claim 1, wherein, when it is possible to confirm frequency band information of a corresponding network from the system information when checking the MCC and MNC a frequency band to be searched is selected according to the frequency band information confirmed from the system information, and the corresponding frequency band is searched, so that network synchronization is performed.

3. The method as claimed in claim 1 or 2, further comprising performing network synchronization by searching for all used frequency bands when the search for the selected frequency band is not normally performed.

4. The method as claimed in claim 3, wherein, when new frequency bands are obtained through the search for all used frequency bands, information for the corresponding frequency bands is updated to the frequency band information table.

5. The method as claimed in claim 4, wherein, when there is no information for the frequency band corresponding to the MCC and MNC checked from the frequency band information table, the bands obtained through the search for all used frequency bands are designated as bands according to corresponding MCC and MNC in order to be stored in the frequency band information table.

6. An apparatus adapted for performing the method according to one of claims 1 to 5.
